Europäisches Patentamt

European Patent Office

Office européen des brevets

(19)

(11) Veröffentlichungsnummer: **0 336 192**
A2

(12) **EUROPÄISCHE PATENTANMELDUNG**

(21) Anmeldenummer: 89104946.2

(22) Anmeldetag: 20.03.89

(51) Int. Cl.⁴: **G01N 33/577 , A61K 49/02**

(30) Priorität: 07.04.88 DE 3811692

(43) Veröffentlichungstag der Anmeldung:
11.10.89 Patentblatt 89/41

(84) Benannte Vertragsstaaten:
AT BE CH DE ES FR GB GR IT LI LU NL SE

(71) Anmelder: **IDT AG FUR IN VIVO DIAGNOSTIK UND THERAPIE**
**Lagerstrasse 107**
**Zürich(CH)**

(72) Erfinder: **Watzek, Carl, Dr.**
**Wichernstrasse 11**
**D-8120 Weilheim(DE)**

(74) Vertreter: **Patentanwälte Deufel- Schön- Hertel- Lewald- Otto**
**Isartorplatz 6 PO Box 260247**
**D-8000 München 26(DE)**

(54) Diagnostikverfahren und Mittel zur Diagnose und Behandlung immunologischer Erkrankungen.

(57) Die Erfindung betrifft ein Verfahren zur Diagnose von erblichen und immunologischen Erkrankungen durch Fluoreszenzmessung biologischer Substanzen, indem man die zu diagnostizierende Substanz, wenn ihr Molekulargewicht über ca. 5000 liegt, mit dem Kondensationsprodukt von Flavoprotein mit Alpha-Liponsäure, das nach Zugabe von Jodacetamid und Dialysieren nach Destillation zurückbleibt, markiert bzw. die zu diagnostizierende Substanz, wenn ihr Molekulargewicht unter ca. 5000 liegt, mit dem weiteren Umsetzungsprodukt des Destillationsrückstandes mit einer Reaktionslösung aus 1,4-Bis(4-methyl-5-phenyl-2-oxazolyl)-benzol und p-Terphenyl in einem organischen Lösungsmittel, dem Maleinsäureanhydrid zugesetzt ist, und das nach mehrstündigem Stehen auf ein etwa neutrales pH eingestellt und in üblicher Weise gereinigt ist, markiert und dann in üblicher Weise, ggfs. nach Abtrennung, die Floreszenzmessung vornimmt oder die zu untersuchende biologische Substanz mit der korrespondierenden markierten Substanz, ggfs. unter Freisetzung des Farbstoffes und ggfs. Übertragung des Farbstoffes auf die korrespondierende Substanz versetzt und dann, ggfs. nach biochemisch üblichem Inkubieren, die Messung durchführt, sowie Mittel zur Durchführung dieses Verfahrens, die derart markiert und ggfs. in an sich üblicher Weise isoliert und ggfs. in Zellen eingebaut sind sowie die Verwendung dieser Mittel auch zur Therapie von immunologischen Erkrankungen durch Bestrahlung der in Lymphozyten eingebauten, fluoreszenzmarkierten biologischen Substanz mit Licht entsprechender Wellenlänge und Kits für das Diagnoseverfahren.

EP 0 336 192 A2

## Diagnostikverfahren und Mittel zur Diagnose und Behandlung immunologischer Erkrankungen.

Die vorliegende Erfindung betrifft neue Fluoreszenzdiagnostikverfahren sowie Mittel zur Verbesserung der medizinischen Diagnostik und Therapie, vor allem auf dem Gebiet der erblichen und viralen Erkrankungen.

Insbesondere betrifft die Erfindung fluorometrische in vitro Bestimmungen von Substanzen in lebenden Zellen und biologischen Flüssigkeiten unter Ausnutzung spezifisch-immunologischer Bindungsreaktionen, insbesondere unter Verwendung in vivo markierter oder kovalent gebundener humaner monoklonaler Antikörper, Antigene oder DNA-Fragmenten.

Es gibt auf diesem Gebiete zahlreiche Nachweisverfahren, die jedoch häufig zu wenig differenziert und/oder zu wenig empfindlich sind.

Erwünscht wäre somit in besonderem Maße eine Verbesserung der medizinischen Diagnostik und Therapie, vor allem auf dem Gebiet der erblichen und viralen Erkrankungen, durch Meßmöglichkeiten in niedrigsten Konzentrationsbereichen der zu bestimmenden Substanz.

Dies wird erreicht, durch die Entwicklung molekularer Diagnosen, die insbesondere der DNA-/RNA-Diagnostik- und Therapie im Bereich bakterieller und viraler Pathogene, bei der Identifizierung von Onkogenen, der Klassifizierung von Leukämien, sowie der Kontrolle von Transplantationserfolgen dienen.

Es wurde gefunden, daß gewisse Farbmoleküle, nämlich das Kondensationsprodukt von Flavoprotein, insbesondere Riboflavin-5-monophosphatnatriumsalz mit Alpha-Liponsäure, das nach Zugabe von Jodacetamid und Dialysieren destilliert wurde, wie dies in der DE-PS 37 05 355 beschrieben ist, in höhermolekulare Proteine, die Molekulargewichte über etwa 5000 haben, z.B. Restriktionsenzyme, bzw. das weitere Umsetzungsprodukt dieses Destillationsrückstandes mit einer Reaktionslösung aus 1,4-bis(4-methyl-5-phenyl-2-oxazolyl)-benzol und p-Terphenyl in einem organischen Lösungsmittel, dem Maleinsäureanhydrid zugesetzt ist, das nach mehrstündigem Stehen auf ein etwa neutrales pH eingestellt und in üblicher Weise gereinigt ist, wie dies ebenfalls in der DE-PS 3705355 beschrieben ist, in niedriger molekulare Proteine, die Molekulargewichte bis etwa 5000 haben, z.B. Polynukleotide, inkorporiert werden können. Diese Fluoreszenzfarbstoffe werden im folgenden Fluoreszenzfarbstoff A (verwendbar bei M.G. über ca. mg 5000) bzw. Farbstoff B (verwendbar bei M.G. bis ca. 5000) genannt. Dies sensibilisiert die DNA und durch die Einwirkung des UV-Lichtes im Anregungsspektrum 360-390 nm wird die DNA nicht inaktiviert. Somit kann das erstgenannte Umsetzungsprodukt von Flavoprotein, insb. Riboflavin5-monophosphatnatriumsalz mit Alphaliponsäure z.B. bei den meisten Antigenen und Antikörpern, z.B. Viren und höhermolekularen Proteinen und DNAS, dagegen das zweitgenannte weitere Umsetzungsprodukt bei niedermolekularen Proteinen und DNAS, z.B. Haptenen und geklonten Fragmenten eingesetzt werden.

Der Vergleich mit virusinfizierten Zellen, die derart mit einem der obengenannten Fluoreszenzfarbstoffe behandelt waren, ergab, daß diese Zellen bei Lichtbehandlung nicht zerstört wurden, aber die Lichtbehandlung zur Inaktivierung der Infektivität der Zelle führte.

Die Zugabe von Serum führte zur Zellvermehrung; dabei wurden die Zellen nicht transformiert, jedoch folgte keine Virusvermehrung. Ähnliche Versuche zeigten, daß das Verhältnis der Inaktivierung von der Anzahl der virusinfizierten Zellen abhängig ist. Nimmt die Zahl der virusinfizierten Zellen zu, erhöht sich die Resistenz gegenüber der Lichtinaktivierung, das heißt jeder infektive Virus provoziert eine neue Synthese irgendeiner präexistenten zellulären DNA.

Bisher konnte die Existenz einer synthetischen viralen DNA in virusinfizierten Zellen nicht eindeutig bewiesen werden. Die bekannen Ergebnisse bezogen sich ausschließlich auf die Verwendung transformierter Zellen.

Weiterführende Versuche bestanden in der Zusammenführung virusinfizierter Zell-DNA mit fluoreszenzmarkierter viraler RNA (in diesem Fall mit Fluoreszenzfarbstoff A) um festzustellen, ob die zwei Einzelstränge dieser zwei Moleküle zu Doppelstrang-Hybrid-Molekülen verschmolzen werden können.

Derartige Bastardisierung ist nur erfolgreich, wenn die DNA-Basensequenz komplementär zur Basensequenz der RNA ist.

Diese Erkenntnis führte zu folgenden Versuchsanordnungen: die DNA-Anteile der RNA-abhängigen DNA-Polymerase (reverse Transcriptase) verfügen über die komplementären Basensequenzen zur viralen RNA.

Die DNA wird fluoreszenzmarkiert, und der DNA-Strang dissoziiert. Der markierte DNA-Einzelstrang wird der unmarkierten viralen RNA zugeführt. Diese Reaktionslösung wird inkubiert, wobei die komplementären Stränge eine Hybrid-Kombination bilden. Anschließend wird die Reaktionslösung in einem Cäsiumsulfat-Dichtegradienten zentrifugiert. Die Hälfte der DNA-Anteile bildet charakteristische RNA-DNA Moleküle.

Durch Bündelung der Sonden mit humanen monoklonalen Antikörpern, vorzugsweise hMak, gewonnen aus Doppelstrang-Hybrid-Molekülen verschiedener Arten, hMAK als Anti-Enzym-Antikörper, humane monoklonale Antikörper mit spezifisch veränderter Genstruktur, sowie humane monoklonale Antkörper, die sich besonders für den Lymphozyteneinbau eignen, ergeben sich mehrere Variationen, je nach dem ob Fluoreszenzfarbstoff A oder B anzuwenden ist. Der Zelleinbau kann variabel erfolgen, entweder durch chemische Synthese mit dem Kaninchen-Anti-Fluoreszenzfarbstoff-Antikörper (A oder B) oder durch in vivo Markierung während der Fusion. Dieser Anti-Fluoreszenz-Antikörper dient zur Kopplung charakteristischer DNA/RNA-Moleküle oder anderer Variationen für den Einbau in antikörperbildende Zellen, wie als Brückenbildner für Mehrfarbenbindungen und zur Koppelung charakteristischer DNA-/RNA-Sonden oder humaner monoklonaler Antikörper verschiedenster Arten.

Somit zeigt die vorliegende Erfindung neue Doppel-Strang-Hybrid-Moleküle gleicher komplementärer Basensequenzen, das Verfahren ihrer Herstellung und die Verwendung als humane monoklonale Antikörper, die durch in vitro Immunisierung und Fusion erhalten werden.

Weiterhin zeigt die Erfindung somit die Herstellung und Verwendung enzymatischer Anti-Antikörper in Assoziation mit Immundefizienz in Lymphozyten.

Die Erfindung betrifft auch Testpackungen, enthaltend die Bestandteile, die für diese Verfahren erforderlich sind.

In der vorliegenden Erfindung bedeutet die Bezeichnung "Transferfaktortherapie" den Einbau spezifischer humaner oder anderer Antikörper mit unterschiedlichen Molekulargewichten in Blutzellen, vorzugsweise humane monoklonale Antikörper, besonders anwendbar bei Patienten mit spezifischen Infektionen.

Die folgenden Beispiele erläutern die Erfindung.

Beispiel 1

Herstellung von in vivo markierten Antikörpern.

Adjuvans I.

ein Reaktionsgemisch aus 0.5 mg/ml Marcol 52 (Standard Oil, Richmond, USA), 0.5 mg/ml Arlacel - A (Atlas Comp. Wilmington, Del.) 5 ml Kochsalzlösung + 2% Tween 80, 0.5 mg/ml D-(+) Trehalose (Sigma) und 0.1 mg/ml Lipid A. wird homogenisiert und in Aliquots von 1 ml abgefüllt.

Adjuvans II

Den Fluoreszenzfarbstoff B gemäß DE-PS 37 05 355, und zwar 50 mmol in 5 ml Kochsalzlösung + 2% Tween 80, lösen. Dieser Fluoreszenzfarbstoff wird als Marker zur Gewinnung von in vivo-fluoreszenzmarkierten Antikörpern im Tier (Kaninchen) verwendet.

Verabreichung:

Adjuvans I 0.85 mg/ml Antigen + 1 ml Adjuvans I - i.m.
Adjuvans II 2 ml Fluoreszenzfarbstoff B i.m.
Titerkonzentrationen nach 3 Wochen.
Adjuvans I 63.096 Adjuvans II 316.227
Adjuvans I entspricht einer ungefähren Determinantengröße in 15 x 20 Angström (A)
Adjuvans einer ungefähren Determinantengröße in 27 x 17 x 6.5 Angström (A).

Anti-Antikörper-Ausfällung und Reinigen.

Aus 50 ml Kaninchenblut wird, wie üblich, Serum gewonnen. Der Antikörper wird nach Angaben des Herstellers (Pharmacia) an 4B-CNBR-Sepharose gebunden und über Affinitätschromatographie gereinigt. Aliquots von 10 μl und 500 μl werden bei -20˚ gelagert.

In entsprechender Weise lassen sich auch enzymatische anti-Antikörper, virale Antikörper, strukturgenetisch veränderte Antikörper oder Fluoreszenz-Antikörper herstellen. Die Fluoreszenz-Anti-Antikörper dienen zur Kopplung von Proteinen für den Einbau in antikörperbildende Zellen. Die aufgeführten Antikörper und ihre Variationen können direkt in vivo markiert gewonnen werden. Sie dienen zum Nachweis von Enzymen, viralen und bakteriellen Pathogenen und anderen Antikörpern oder Antigenen in biologischen Flüssigkeiten.

Beispiel 2

Bestimmung von Peptiden und deren Analogen durch Fluoreszenztechnik.

Die Lymphozyten-Zellsuspension kurz vor dem Test anfertigen. Ein Volumen von Lymphozyten wird in einem Zentrifugenglas 3x mit isotonischer Kochsalzlösung gewaschen und zentrifugiert. Das Lymphozyten-Volumen wird je nach gewünschter Konzentration verdünnt.

| Beispiel: | Zellvolumen | Endvolumen/Verdünnung | | |
|---|---|---|---|---|
| | | 2 % | 5 % | 10 % |
| | 0,1 ml | 5 ml | 2 ml | 1 ml |
| | 0,5 ml | 25 ml | 10 ml | 5 ml |
| | 1,0 ml | 50 ml | 20 ml | 10 ml |

Die Lymphozyten-Zellsuspensions-Verdünnung richtet sich nach der Konzentration der zu bestimmenden Antikörper.

Testablauf:

Zellsuspension auf saubere Objektträger ausstreichen und lufttrocknen. (Keine Fixativ-Reagenzien benutzen), fluoreszenzmarkierte humane monoklonale Adenosin-Deaminase-Antikörper (ADA) und humane monoklonale Purin-Nukleotid-Phosphorylase-Antikörper (PNP) mit PBS-Puffer, pH-7,2 1:10 verdünnen und auf die Probe auftropfen. 30 Minuten in feuchter Kammer bei 37° C inkubieren. Danach Objektträger 1x sehr sanft mit PBS-Puffer, pH-7,2 abspülen, Objektträgerkanten abtupfen und mikroskopisch ablesen.

Wasserunlösliche Nitrozellulose-Plättchen (Durchmesser 5 mm) werden fest in flachbödige Vertiefungen einer Mikrotiterplatte eingefüllt. 100 μl Patientenserum wird auf die Plättchen aufgetropft und über Nacht abgedeckt und bei Raumtemperatur getrocknet. Die trockenen Zellulose-Plättchen werden mit 200 μl Blockierungslösung (12%ige Molke in 0.05 M Natriumzitratpuffer, pH 5.5 und 0.1 % Natriumazid, 1 Vol. Molke + 4 Vol. Aqua bidest) überflutet, abgedeckt und 30 min bei 37° C inkubiert. Die Blockierungslösung wird abgesaugt und der Rückstand wird mit 200 μl Waschlösung (0,9 % NaCl mit 1% Tween 20) 10 min bei Raumtemperatur stehengelassen. Danach wird die Waschlösung abgesaugt. Die Mikrotiterplatten werden auf saugfähigem Papier ausgeklopft.

Auf Plättchen der Reihen 1 - 3 werden 100 μl fluoreszenzmarkierte humane monoklonale Anti-PNP-Antikörper, auf Plättchen der Reihen 4 - 6 werden 100 μl fluoreszenzmarkierte humane monoklonale Anti-ADA-Antikörper, und auf Plättchen der Reihen 7 - 9 werden 100 μl fluoreszenzmarkierte humane monoklonale Peptid- oder Neuropeptid- oder Neutropeptid Antikörper (Kleinzell-Karzinomzellklon) und auf die Plättchen 10 - 12 werden 100/μl fluoreszenzmarkiertes humanes IgG-Kontrollserum aufgetropft und 1 h bei 37° C inkubiert.

Nach Waschen wie vorher werden 50μl Stoplösung (0,1 N NaOH) auf die Plättchen pipettiert und es wird sofort visuell abgelesen. Das Endresultat ist nach Lufttrocknung bei Raumtemperatur photometrisch bei 405 nm und fluorometrisch im Anregungsspektrum 360 bis 390 nm (blau) und im Emissions-Spektrum 405 nm (gelb), 425 bis 450 nm (grün) und 490 bis 550 nm (rot) ablesbar. Die einzelnen Farbbanden zeigen die Farbenvielfältigkeit der Fluoreszenzfarbstoff-Komponenten A und B.

Gefrierschnitte und andere fixierte Gewebematerialien können nativ fixiert hergestellt werden. Antigene, die in bestimmen Geweben oder Einzelzellen nachzuweisen sind, müssen ohne Verlust der Antigenität fixiert werden. Für intrazytoplasmatische Globuline eignet sich eine Fixierung in 5%iger Essigsäure in absolutem Äthanol bei -20° C besonders gut. Bei Membranfluoreszenzverfahren werden die Präparate zur Dokumentation mit einer einfachen Fixierung in 95%igem Äthanol empfohlen.

4

Die Einwegküvetten bzw. die Bechergläser werden mit PBS-Puffer gefüllt. Das Präparat wird zunächst mit zwei Pinzetten durch den Puffer im Glas leicht geschwenkt und dann für 30 min in ein mit Puffer gefülltes Küvettenglas gestellt. Die Waschflüssigkeit wird nach 15 min erneuert. Dann läßt man die Waschflüssigkeit auf Saugpapier abtropfen und legt das Präparat in die feuchte Kammer. Es werden 1 bis 2 Tropfen fluoreszenzmarkierte humane monoklonale Antikörper ADA und/oder fluoreszenzmarkierte humane monoklonale Antikörper PNP aufgebracht und 30 min bei 37° C inkubiert. Dann wird wie vorher gewaschen und das Präparat danach 90 min im Küvettenglas stehengelassen. Dann läßt man die Präparate trocknen. Man legt die Deckgläser auf Filterpapier aus und bringt je einen Tropfen fluoreszenzfreies Glyzerin (9 Teile Glyzerin p.a./1 Teil PBS-Puffer) auf. Die Präparate werden mit den Schnitten nach unten leicht auf Deckgläser gepreßt und überschüssiges Glyzerin wird mit Filterpapier abgesaugt und das Deckgläschen mit Nagellack fixiert, indem der Rand mit Lack umstrichen wird. Dann wird mikroskopisch ausgewertet.

Für den Fluoreszenz-HIV-Virusnachweis unterschiedlicher HIV-Linien dürfen Gewebe, Lymphozyten, Lymphknoten und anderes Biopsiematerial, also auch anderes Gewebematerial nicht in Paraffin oder Gelatine eingebettet werden, da diese störende Eigenfluoreszenz aufweisen können. Die auftretenden Interferenzen von Eigenfluoreszenz der Kombination Einbettungssubstanz/Viren führen zu Irritationen bei der Auswertung. Die Durchführung ist identisch mit der Testbeschreibung im folgenden Beispiel 3. Nur die Inkubationszeit und Temperatur beim Nachweis von HIV-Antigenen und HIV-Antikörpern verschiebt sich von 30 min bei 37° C auf 45 min bei 40° C.

Es können insbesondere Neuropeptide (mit Farbstoff B) und Enzyme (mit Farbstoff A) mit hochsensitiver Fluoreszenztechnik zum Nachweis von Auslösern oder Begleitern neoplastischer Prozesse in Assoziation mit erworbener oder kombinierter Immundefizienz in Lymphozyten, Serum, Plasma oder anderen biologischen Flüssigkeiten, in Gefrierschnitten und anderen Gewebepräparationen bestimmt werden.

## Beispiel 3

Bstimmung von Antigenen und Antikörpern geringer Immunreaktivität zum Nachweis verschiedener HIV-Linien in Serum, Plasma, Urin mit hochsensitiver Fluoreszenz und humanen monoklonalen DNA-RNA-Hybridkombination Antikörper (HIV).

Mikrotiterplatten, Mikrotiter-Strips, Nitrozellulosestreifen und -plättchen, ausgestanzte Objektträger, Reagenzröhrchen, Mikrotuben, Plastikkugeln und anderes Material werden mit hitzeinaktivierten Virusantigenen beschichtet, getrocknet und vor Gebrauch 2 mal mit 200 µl Waschlösung (Tris-NaCl-Puffer, pH 7,5 mit 1% Tween 20/Verdünnung 1:9) gewaschen. 100 µl verdünntes Patientenserum (10 µl Serum + 100µl Blockierungslösung) werden in die Vertiefungen pipettiert, abgedeckt und 45 min/37° C im Wasserbad oder in einer feuchten Kammer inkubiert. Danach wird 2 x mit Waschlösung gewaschen und auf saugfähiges Papier abtropfen gelassen. 100 µl fluoreszenzmarkierter viraler humaner monoklonaler Antikörper werden in die Vertiefungen pipettiert, die Platte wird mit Folie abgedeckt und 45 min bei 37° C inkubiert. Nach Inkubation wird abgesaugt, die Platte bzw. Strips umgekehrt auf saugfähiges Papier gelegt und trocknen gelassen. Danach erfolgt Zugabe von 50 µl Stopplösung (1 N NaOH) und es wird gemessen.

Bestimmung verschiedener HIV-Linien und verschiedener HIV-anti-Antikörper sowie anderer viraler Antigenlinien und anderer viraler anti-Antikörper mit hochsensitiver Fluoreszenztechnik in verschiedenen Trägersystemen wie beschrieben für den Nachweis obiger Antigen-Antikörper Variationen in niedrigen Konzentrationsbereichen in einem Testsystem.

Die Trägersysteme werden mit 5 µl Antigen beschichtet und luftgetrocknet. Die trockenen Trägersysteme werden mit der Blockierungslösung 45 min bei 37° C im Wasserbad oder in einer feuchten Kammer inkubiert, abgesaugt und 2 x mit Pufferlösung gewaschen. In die Vertiefungen der Reihen 1-9 werden 100µl unbekannte Patientenseren (Verdünnung 1:50) pipettiert. Die Reihen 11-12 werden mit 100 µl humanen monoklonalen Antikörper 1:100 verdünnt (10µg/100µl) beschickt, abgedeckt und 45 min bei 37° C im Wasserbad oder in einer feuchten Kammer inkubiert.

Nach dreimaligem Waschen und Abtrocknen auf saugfähigem Papier werden 100 µl fluoreszenzmarkierte spezifische humane monoklonale HIV-Antikörper in alle Vertiefungen pipettiert. Nach erfolgter Inkubation (45 min/37° C) und dreimaligem Waschen erfolgt die Zugabe von 50µl Stoplösung (1 N NaOH) und einmaliges Waschen. Bei visueller Betrachtung im UV-Licht zeigen die positiven Reaktionen ein intensives Blaulicht, variabel je nach Antikörperkonzentration im Bindungskomplex. Die Endresultate liegen nach Trocknung bei Raumtemperatur über Nacht vor. Sie können visuell, mikroskopisch, photometrisch oder fluorometrisch bestimmt werden.

Resultate auf Durchführbarkeit des Testverfahrens. Die Intensität der Färbung wird anhand einer relativen Abgradierung in vier Stufen für den visuellen Bereich ausgedrückt.

0 = keine Färbung, + = kaum sichtbare Färbung,

+ + = blasse Färbung, + + + = helle Färbung,

+ + + + = intensive Färbung.

Klar positive Ergebnisse zeigten sich bei einer Antigenverdünnung von $10^{-3}$. Die Farbintensität korreliert mit der gebundenen Antigenmenge.

Unspezifische Bindungsreaktionen treten weder im unverdünnten Antigen, noch in Verdünnungen bis $10^{-6}$ auf.

Die Serumverdünnungen von 1:100 zeigen sich für alle Seren im Test optimal.

Antikörperverdünnungen von 1:800 sind ausreichend für den Antigennachweis. Die Meßgrenze durch zusätzliche Verdünnungen im Testablauf liegt zwischen $10^{-14}$ und $10^{-17}$ (pmol-fmol).

Die beschriebenen Testverfahren für HIV-Nachweise zeigen zwei Charakteristika auf: Die Gewebeuntersuchung weist den aktuellen Grad der Infektivität aus, der immunologische Assay weist die Summe von Virusantigenen, unabhängig von seinen biologischen Aktivitäten nach.

Beispiel 4

Fluoreszenzverfahren zum Nachweis verschiedener HIV-Antigene in Gameten.

Männliche und weibliche Geschlechtszellen (Eizellen, Spermien) werden ausgestrichen und 45 min luftgetrocknet. (Kürzere Trocknung führt zum Verlust des Materials durch Ablösung während der Waschvorgänge). Zur kompetitiven Hemmung unspezifischer Bindungen wird eine Verdünnungslösung (PBS-Puffer, pH 7,0 mit Zusatz von 2-5% bovinem Rinderalbumin/Behring) hergestellt. Mit einer Pasteurpipette wird die Serumverdünnung auf das Präparat gebracht. Die Präparate müssen vollständig überflutet sein.

Die Präparate werden 30 min in einer feuchten Kammer bei Raumtemperatur inkubiert. Dann erfolgt Waschen mit PSB-Puffer pH 7,0. Nach dem zweiten Eintauchen wird der Puffer gewechselt und die Präparate werden auf saugfähigem Papier trocknen gelassen und danach werden 1 bis 2 Tropfen mit fluoreszenzmarkierten. antigenspezifischen humanen monoklonalen HIV-Antikörper auf das Präparat aufgebracht und es wird weitere 60 min bei Raumtemperatur inkubiert. Dann wird mit PBS-Puffer gewaschen und 60 min im Küvettenglas stehengelassen. Nach Abtrocknen werden die Deckgläser mit einem Tropfen fluoreszenzfreiem Glyzerin versehen, abgedeckt und überschüssiges Glyzerin wird nach leichtem Anpressen der Deckgläschen mit Filterpapier abgesaugt. Evtl. Luftblasen verursachen Autofluoreszenz und sind daher zu vermeiden, da der Bildkontrast wesentlich herabgesetzt wird. Die Deckgläser werden dann mit Nagellack befestigt, und die Präparate können danach visuell mit UV-Lampe oder mikroskopisch ausgewertet werden.

Beispiel 5

Herstellung von fluoreszierenden DNA- und RNA-Sonden.

Desoxiribonukleinsäuren (DNA), Ribonukleinsäure (RNA), ribosehaltige Nukleotide, Nukleoside, Mono-Polynukleotide, sowie RNA-Matritzen-(nRNA, rRNA, tRNA, hnRNA) RNA/DNA-Polymerasen, Proteinbruchstücke, aus den nRNA partiell synthetisierten Peptiden, werden wie folgt behandelt.

Reagenzien/Verfahren

4 M Natriumchlorid

10% Natriumdodecylsulfat (NDS)

10 N NaOH

```
100 µM Deoxyadenosin-5-triphosphat (dATP)⎤ mit Fluores-
100 µM Deoxyguanin  -5-triphosphat (dGTP) ⎮ farbstoff B gem.
100 µM Deoxycytosin-5-triphosphat(dCTP)   ⎬ DE-PS 3705355
100 µM Deoxythymidin-5-triphosphat(dTTP)  ⎦ fluoreszenz-
                                              markierte
                                              Substanzen
```

2 M Tris-HCl, pH - 7,5
0,25 M EDTA, pH - 7,5
1 M Hepes, pH - 7,0 (Na$^+$)
1 M Magnesiumchlorid
2 M Natriumacetat, pH - 6,0
2 M Kaliumchlorid

Gebrauchslösung I :

2 mg Polyvinylpyrrolidon (PVP 360), 2mg Ficoll 400, 2 mg Rinderalbumin mit 100 ml Aqua bidest.ansetzen. 20 ml NNC (3 M NaCl, 0,3 M Natriumcitrat) und 50%ige Sucrose zugeben.

Gebrauchslösung II:

Redestilliertes Phenol + Chloroform + Isoamylalkohol (25 : 24 : 1) saturiert mit 20 mM Tris, pH -7,5
1 M Tris-HCl, pH 8,0
Gelatine 5 mg/ml
0,25 M EDTA, pH - 8,0
10% Triton X - 100
100 ml TEA-Lösung: 0,4 M Tris, 0,2 M Natriumacetat, 0,18 M Natriumchlorid, 0,02M EDTA-Na$_2$, pH - 8,05 mit Eisessig, 0,8 % Agarose in 10 ml TEA-Lösung.
Formamid-Lösung: Formamid 100%-entionisiert, 3 mM EDTA, pH -7,5, 0,1% Diaethyloxydiformat (DEP): 5 mg Amberlite MB-1 zu 100 ml Formamid zugeben und abgedeckt 1 h rühren. Amberlite ausfiltern. Nach Zugabe von 3 mM EDTA und 0,1 % DEP im kochenden Wasser ca. 5 min stehen lassen, abkühlen.
Dowex 50 Vorbereitung: 100 ml gewaschenes Dowex 50 nochmals in 500 ml 2 M NaOH waschen, 2 Waschungen mit Wasser, Waschung mit 0,25 N HCL 5 x Waschung mit Wasser, Resuspendierung in H$_2$O und pH - 8,0. Gepackte Dowex-Säule mit 0,1 M Tris-HCl äquilibrieren.
Salmon sperm DNA-(Sigma) 1-10 mg/ml : 1 mg DNA in 10 mM Tris-EDTA, pH - 7,5 lösen, 5 µg Fluoreszenzfarbstoff A unter langsamen Rühren zugeben und 2 /ul/ml 0,3 N NaOH zugeben. 20 min im Heißwasserbad inkubieren, Abkühlen im Eisbad und dann 2 mM Tris pH - 7,5 zugeben bis eine Endlösung von 0,1 M erreicht ist. Dann 2 M Natriumacetat pH - 6,0 bis zur 0,2 M Endlösung zugeben, bei 10.000 g/10 min zentrifugieren, mit 70%igem Ethanol waschen und wieder zentrifugieren. Niederschlag in 10 ml TRIS - EDTA lösen, auf pH - 7,5 einstellen und 100 ug/ml Proteinase K zufügen.
Dann erfolgt Zugabe von l0%iger NDS-Lösung bis die Konzentration der Endlösung 0,5% beträgt. Das Präparat wird 2 h ins Eisbad gestellt, dann bei 2000g/15 min zentrifugiert, in 1mm Tris,pH - 7,5 resuspendiert und gegen Tris 1 mM dialysiert.
E.Coli-DNA 1 - 10 mg/ml
Hefe-RNA, 10 mg/ml
Chloramphenicolpulver
Nährlösung: 10 mg Trypton, 5 mg Hefeextrakt, 5 gm NaCl, 1 mg Glukose, 0,1 ml 10 N NaOH in 1000 ml Aqua bidest auflösen, Tetracyclin-Platten: 1,5%iger Nähragar in 5%/L Boullion lösen, auf eine Konzentration von 25 µg/ml Tetracyclin einstellen. In Petrischalen abfüllen.
Lytische Lösung: 0,3 % Triton X-100, 0,187 M EDTA, pH - 8,0 0,15 M Tris-HCl pH-8,0 auf 100 ml.
Ribonuklease, 10 mg/ml in 10 mM Tris-HCl, pH - 7,5 Proteinase K 10 mg/ml in 10 mM Tris-HCl, pH 7,5
DNA - Polymerase I / RNA-abhängige DNA - Polymerase Restriktions-Endonuklease/Mbo II oder Äquivalent (z.B. Mbo III)
95%iger Ethanol - 20° C,
70%iger Ethanol - 20° C,

HIND III digest DNA 0,1 - 0,5 mg/ml
Nitrozellulosepapier Porengröße 0,45 μm
E.Coli mit Plasmiden als Carrier für Alpha-Globin, Beta-Globin oder Gamma-Globin Gene.
Lysozym 5 mg/ml in 25 mM Tris-HCl, pH - 8,0

## Isolierung von DNA/Plasmid-DNA

1) Tetracyclin-Platten in 10 ml Nährlösung über Nacht bei 37° C inkubieren.

2) Überimpfung der 10 ml Nährlösung in 1 Liter Nähr-Stammlösung. Unter ständigem Rühren 5 h bei 37° C inkubieren bis O.D. (opt. Dichte) von 650 nm erreicht ist.

3) Zugabe von Chloramphenicolpulver bis zu einer Konzentration von 170 μg/ml und weitere Übernachtinkubation unter Rühren.

4) Kulturmedium in 150 ml Flaschen füllen und bei 10.000g/20 min zentrifugieren, abdekantieren und Niederschlag in Ethanol-Trockeneis über Nacht einfrieren.

5) Niederschlag in 150 ml Flaschen mit 15 ml kaltem 50 mM Tris-HCl, pH - 8,0 versetzt mit 25 % Sucrose, resuspendieren.

6) Zugabe von 3 ml Lysozym, 5 min Eisbad.

7) 1,5 ml 0,25 M EDTA, pH - 8,0 zugeben, 15 min Eisbad.

8) 15 ml kalte lytische Lösung zugeben, sanft rühren und 30 min im Eisbad halten.

9) in 50 ml Röhrchen überführen und bei 34.000 g/l h bei 4° C zentrifugieren.

10) Überstand in 250 ml Erlenmayerkolben dekantieren, mit demselben Volumen Wasser und 5 μl DNA (Sigma) auffüllen und 10 min bei Raumtemperatur stehen lassen.

11) DNA mit demselben Volumen Gebrauchslösung II unter 10-minütigem leichten Schütteln extrahieren, 10 min in Eisbad stellen, zentrifugieren, wässrige Phase abtrennen und mit 2 M Acetat auf 2,0 M einstellen.

12) 2 Volumen kalten 95%igen Aethanol zugeben, leicht mischen und bei - 20° C über Nacht DNA ausfallen lassen.

13) Bei 12.000 g/30 min zentrifugieren (4° C).

14) DNA-Niederschlag in 2-5 ml 50mM Tris-HCl, pH - 7,5 mit 0,5 M NaCl auflösen auf eine Säule (2,5x40 mm) mit A 50 Agarose ziehen. DNA-Fraktionen mit demselben Volumen kalten 95%igen Aethanol auffüllen, mischen und über Nacht einfrieren. Danach bei 12.000 g oder mehr 1 h zentrifugieren. Präzipitierte DNA in 2,1 ml oder mehr an 50 mM Tris-HCl, pH - 7,5 auflösen. In jede 2,1 ml gelöste DNA 2,2 g Caesiumchlorid zugeben und lösen und 0,15 ml Fluoreszenzfarbstoff A (1 /ug/ml) zugeben.

15) Je 2,8 ml DNA in Zellulosenitrat-Röhrchen mit einer Schicht sterilem Mineralöl auffüllen und mit 100.000 g (im SW 50.1 Rotor bei 36.000 Upm) bei 20° C 48 h zentrifugieren.

16) Bandenprüfung unter UV-Licht. Die untere Bande ist DNA, die obere Bande ist chromosal. Untere Bande vorsichtig mit einer Kanüle in ein Zentrifugenröhrchen überführen, und über eine Pasteurpipette mit 1-2 ml Dowex oder Sephadex versetzen und mit einer Waschlösung von 0,01 mM Tris-HCl, pH - 8,0 und 0,5 M NaCl waschen.

17) DNA gegen 50 mM Tris-HCl, pH - 7,5 und 0,3 M NaCl 2 bis 3 h dialysieren, mit 2 Volumen 95%igen Aethanol auffüllen und mehrere Stunden bei -20° C DNA ausfallen lassen, danach zentrifugieren, Überstand entfernen, Niederschlag mit 25 mM Tris-HCl, pH 7,5 und 0,1 mM EDTA lösen, in Aliquots abfüllen und bei -20° C aufbewahren.

## Schneiden der DNA

100 μg DNA mit Mbo II nach Angaben der Hersteller inkubieren.

1) Gelöste DNA (500 μl/5 mM Tris, pH - 7,5, 0,1 mM ETDA und 250 μl 50%ige Sucrose, vermischt mit 1 ml Fluoreszenzfarbstoff A = 1 μg/ml) in Agargel Einschnitte verbringen. Durchführung der Agarose-Elektrophorese nach bekannter Technik (Phast-System Pharmacia).

2) DNA gegen 1 mM Tris, pH - 7,5 mit 0,1 mM EDTA dialysieren und lyophilisieren.

3) Auflösung der DNA in 5 mM Tris, pH - 7,5 und 0,1 mM EDTA. Ergibt 50 - 100 ug/ml konzentrierte DNA.

## Nick Translation der Globin Gene

1) In einem Röhrchen werden 15 μl fluoreszmarkiertes dATP, dGTP, dCTP, dTTP, 10 μl Puffer 500 mM Tris-HCl, pH - 7,5, 50 mM MgC, 100 mM ß-Mercaptoaethanol und 500 μg/ml Gelatine in Wasser bis zu einem Volumen von 97 μl aufgefüllt. Nach Zugabe von 3 μl DNA-Polymerase I oder RNA-abhängige DNA-Polymerase (Revers Transkriptase) wird 3 h bei 20° C inkubiert.

2) 0,4 ml Stoplösung-(12,5 mM EDTA, pH-7,5, 0,625 % Natriumdodecylsulfat, 125 μg/ml E.Coli DNA) zugeben, auf 65° C erhitzen (10 min).

3) Zugabe von 500 μl Gebrauchslösung II, schütteln und wenige Minuten in Eisbad stellen. In Eppendorfzentrifuge 12.000 g/3 min zentrifugieren. Wässrige Schicht entfernen, dem Rückstand einen Tropfen 50%iger Sucrose zugeben und auf eine Sephadex-Säule G-50 bringen, mit 10 mM Tris-HCl, pH - 7,5 und 0,5 mM EDTA aequilibrieren. Bei einer Pumpenleistung von 8 ml/h werden 2 Minutenfraktionen in Mikroröhrchen (Eppendorf) gesammelt.

4) Zu 5 ml 0,6 N NaOH werden 50 μg/ml DNA, Restriktions-Enolasen, Nukleinsäure modifizierende Enzyme, RNA-abhängige DNA-Polymerasen und andere Komponenten mit bestimmten Erkennungssequenzen in Mengen von 50 μg/ml oder in Mengen von 10 - 500 Einheiten zugegeben und es wird 1 h bei 37° C inkubiert. Danach wird mit konzentrierter HCl neutralisiert.

5) Dazu werden 2 Vol. 95%iges Aethanol gegeben und es wurde im Eisfach über Nacht auspräzipitiert. Dann wird 2 - 3 min in der Eppendorfzentrifuge zentrifugiert, mit 70%igem Aethanol gewaschen und die Hybridisierungslösung im Vakuum getrocknet.

6) 0,4 ml der getrockneten Hybridisierungslösung werden in 5 mM Tris, pH - 7,5, 0,1 mM EDTA gelöst, die Lösung wird 5 min in kochendes Wasser gestellt, danach wird in Eis abgekühlt und das Präparat in Aliquots abgefüllt und bei -20° C aufbewahrt.

## DNA - Extraktion

1) Gewinnung von Leukozyten:
20 ml Frischblut werden mit 6,5 ml in einer Mischung von 5%igem PVP (Polyvinylpyrrolidon) und 3 % Natriumcitrat gemischt und 30 bis 45 min bei 37° C inkubiert. Die Plasmaphase über den sedimentierten Erythrozyten wird abgehoben und bei 1000g/10 min zentrifugiert. Der Niederschlag wird 2 mal mit 0,9%igem NaCl gewaschen.

2) Proteinase K-Auflösung:
In einem 125 ml Kolben werden Leukozyten mit 2,5 ml 10%igem Natriumdodecylsulfat, 0,5 ml Proteinase K und 0,05 M Tris-HCl, pH - 7,5 versetzt und mit 0,1 M NaCl + 1 mM EDTA auf ein Endvolumen von 50 ml gebracht. Das Gemisch wird bei 50 - 55° C rotierend im Wasserbad über Nacht inkubiert

3) Phenolextraktion:
Bei Raumtemperatur wird 50 ml Gebrauchslösung II zugegeben. Dann wird gut 10 min geschüttelt, 10 min ins Eisbad gestellt und danach 15 min bei 3000 - 4000 g zentrifugiert.

4) Die obere wässrige Phase wird abgehoben und 2 M Natriumacetat purum (Fluka) auf 0,2 M/pH - 6,0 eingestellt. Danach wird mit 2 ml 95%igem Aethanol gemischt und 1 ml Fluoreszenzfarbstoff B auf pH - 6,0 (1 μg/ml) zugegeben. Das Präparat wurde über Nacht bei -20° C aufbewahrt und anschließend bei 3000 - 4000 g 15 min zentrifugiert. Der Niederschlag wird mit 70%igem Alkohol gewaschen.

5) Der Niederschlag wird in 10 ml 10 mM Tris-HCl, pH - 7,5 + 10 mM EDTA gelöst, es werden 100 μl RNAse zugegeben und dann wird 2 h bei 37° C inkubiert. Danach wird 0,5 ml 10%ige Natriumdodecylsulfatlösung und 100 μl Proteinase K zugegeben und 1 h bei 50 - 55° C inkubiert. Schließlich wird 10,7 ml Gebrauchslösung II zugegeben und es wird damit extrahiert.

6) Der Niederschlag wird gut filtriert, mit 1 bis 2 ml 1mM Tris, pH - 7,5 vermischt und in 0,1 mM EDTA aufgelöst, 2 Tage bei mehrmaligem Wechsel der Dialyselösung ein 1mM Tris, pH - 7,5 + 0,1 mM EDTA dialysiert. Die Prüfung erfolgt durch O.D.260/280. Das 260/280 Verhältnis sollte höher als 1,6 sein. Bei zu geringem Wert muß die Arbeitsweise beginnend mit der Behandlung mit Proteinase K der Stufe 2 nochmals wiederholt werden. Berechnung: μg/ml DNA = O.D 260 x Verdünnung x 50.

## DNA-Auflösung mit Restriktions-Endonukleasen

DNA Auflösungen werden nach Angaben der R-Endonukleasen-Lieferanten normalerweise mit 10 μg DNA, vermischt mit 100 μg/ml Gelatine in 6 mM ß-Mercaptoethanol vorgenommen. Das 200 μl-Gemisch wird mit einem 20-fachen Überschuß von MBOII, ECO RI, HPa I, BAMH I, und anderen üblichen Nukleinsäure modifizierenden Enzymen sowie Fluoreszenzfarbstoff A bei 37° C/5 h inkubiert. Nach Inkuba-

9

tion mit den gleichen Volumen Gebrauchslösung II und Aethanol ausfällen, 2-3 min zentrifugieren, Niederschlag mit 70%igem Alkohol waschen und im Vakuum-Exsikkator trocknen. Die DNA-Proben werden in 2,8 ml (oder mehr 50 mM Tris-HCl, pH-7,5 gelöst. Zu jeder 2,8 ml Restriktions-Endonukleasen-Probe werden 1,5 g CsSO und 0,20 ml einer 0,1 %igen Fluoreszenzfarbstofflösung A zugegeben. UZ-Röhrchen werden mit 3 ml Probe beschickt und mit einer sterilen Mineralölschicht aufgefüllt. Im SW 50.1 Rotor/Beckman-Ultrazentrifuge bei 100.000xg bei 20°C, 48 Std. zentrifugieren. Danach die Bandenpositionen unter UV-Licht bestimmen. Die Bande 3 entspricht den charakteristischen DNA-RNA-Hybridmolekülen. Die Bande 3 mit einer Druckflügelkanüle anpunktieren und die Bande in ein Zentrifugenglas überführen. Pro ml Hybridmolekül 1 ml DOWEX 50 in 0,1 M Tris-HCl, pH-8,0 lösen, in Pasteur-Pipetten füllen, setzen lassen, auf diese Pipettensäule DNA/RNA-Hybridmoleküle aufbringen und mit 0,5 M NaCl langsam den überschüssigen Fluoreszenzfarbstoff A entfernen. Hybridome gegen 50 mM Tris-HCl, pH-7,5 und 0,3 M NaCl 2 Std. dialysieren, danach mit 2 Vol. 95 %igem Äthanol bei -20°C mehrere Stunden ausfällen. Präzipitat in Eppendorfzentrifuge zentrifugieren, Niederschlag in 25 mM Tris-HCl, pH-7,5 + 0,1 mM EDTA auflösen und in Aliquots abfüllen und aufbewahren.

Elektrophorese

Nach dem neuen Phast-System (Pharmacia/Uppsala) wurde ein Elektrophoretisches screening mit den Systemen SDS-PAGE (Silberfärbung), NATIVE PAGE (Coomassiefärbung), IEF-Immunelektrophorese und Titrationskurven-Elektrophorese durchgeführt. Jede der Elektrophoresesysteme sind auf eigenen Elektrophorese-Gel-Plättchen vorgenommen worden, ein Schneiden der Gele fällt weg. Die Gel-Platten sind dokumentierbar, fotografierbar und im UV-Filter-Bereich anwendbar.

Transfer von DNA auf Nitrozellulosefilter

Das Agarose-Gel läßt man 2 h in 1,5 M NaCl/0,5 M NaOH bei Raumtemperatur vollsaugen und dann weitere 3 h in 3M NaCl/0.5 M Tris-HCl, pH - 7,5, erneut vollsaugen. Dann werden Nitrozellulosefilter ausgeschnitten und 6 mal mit 3 M NaCl + 3 M Natriumcitrat befeuchtet. Whatman No. 1 Filterpapir wird ausgeschnitten und wiederum mehrmals mit der obigen Lösung befeuchtet und das Filterpapier wird auf das Nitrozellulosefilter aufgelegt. Dann wird in Folien verpackt und mit Klebeband verschlossen. Das Präparat wird 40 - 48 h bei Raumtemperatur aufbewahrt, dann wird das Nitrozellulosefilter entfernt und mit Whatman No. 1-Papier 2 h bei 80°C im Vakuumofen getrocknet.

Hybridisierung

1) Eine trockene Nitrozellulosefilterplatte wird mit einer Hybridisierungslösung vollgesaugt. (50% Formamid, 0,05 M Hepes, pH - 7,0, Gebrauchslösung I, NNC-Lösung = 3 M NaCl, 0,3 M Natriumcitrat, 0,2 mg/ml DNA(Sigma) und 0,15 mg/ml Hefe-RNA). Das Gesamtvolumen der Hybridisierungslösung beträgt 5 ml. Die Filter werden in Folie gewickelt und 2 h bei 41°C inkubiert.
2) Für die Hybridisierung werden 15 μl/ml der Globingene in die Hybridisierungslösung pipettiert. Die Filter werden auf eine neue Folie gelegt und mit einer Pasteuerpipette wird die Globinlösung verteilt. Die Folie wird verschlossen und bei 41°C 3 Tage inkubiert.
3) Man läßt die Filter 1 h mit 2 Vol. NNC-Lösung und 1 Vol. Gebrauchslösung I (ca. 200 - 300 ml) vollsaugen, wäscht sie danach vorsichtig und läßt sie nochmals 1 -1 1/2 h mit 1 Vol. NNC-Lösung und 1 Vol. Natriumdodecylsulfat-Lösung vollsaugen. Dann spült man mit 0,1 % NNC-Lösung nach. Die Filter werden bei Raumtemperatur getrocknet und unter UV-Licht fotografiert.

In entsprechender Weise kann die Herstellung von fluoreszierenden DNA- und RNA-Sonden und charakteristischer DNA/RNA-Moleküle, von polymorphen Restriktionsenzymfragmenten und deren Komponenten zur Aufspürung und Charakterisierung von Gendefekten, Onkogenen und verstreuten Genen in Immunglobulinen, von Globingenen, RNA-abhängiger DNA-Polymerase, Restriktions-Endolasen und anderen Verbindungen zur Therapie und Diagnstik immunologisch genetisch bedingter Erkrankungen erfolgen.

Beispiel 6

Herstellung von humanen monoklonalen Antikörpern (hMAK)

Humane monoklonale Antikörper aller Art können unter Anwendung der in vitro Immunisierung und Fusion erhalten werden. Die humanen monoklonalen Antikörper können entweder als in vivo markierte oder durch chemische Kopplung kovalent gebunden zum Nachweis von Antikörpern und Antigenen in biologischen Flüssigkeiten verwendet werden. Die humanen monoklonalen Antikörper eignen sich zum Einbau in Blut- und andere Zellen für die Anwendung der Transferfaktortherapie bei spezifischen Infektionen oder zur spezifischen Klonherstellung.

In vitro Immunisierung:

Aufbereitung von Lymphozyten, Fibroblasten, Makrophagen, dendritischen Makrophagen oder anderen Lymphozyten-, oder Blutzellarten für die in vitro Immunisierung.

1) Blutzellmaterial wird nach Zentrifugation (1000 g/5 min) 2 mal mit 20 ml serumfreien RPMI-Medium 1640 gewaschen und mit sterilem Glasstab resuspendiert. Nach Zellzählung im humanen Serum Medium (9 Vol Human-Serum/Sigma, 1 Vol. 4,5 % EDTA + 0,3 % NaOH) wird unter Zugabe von $5 \times 10^{-5}$ M Mercaptoäthanol in Gewebekulturflaschen überführt und 4 Tage in 5%iger feuchter $CO_2$-Kammer inkubiert.

2) Myeloma, Plasmozytome, Milz, Tumore, Drüsen und Zell-Linien aller Arten humanen Ursprungs werden von Fremdgewebeteilen befreit und durch ein Sieb in eine Petrischale mit 10 ml serumfreien RPMI-Medium gepreßt. Die Zellen werden durch Zentrifugation (50 g/5 min) 2 mal mit 20 ml serumfreien RPMI-Medium gewaschen und auf eine Zelldichte von $5 \times 10^6$ Zellen/ml im RPMI-Medium eingestellt. Dann erfolgt Zugabe von 1 ml 0,1 mM Pyrrolidoncarbonsäure (PCA) und 1 ml 0,01 mM CoA-S-S-Glutathion, verknüpft mit 1 μg/ml Fluoreszenzfarbstoff A. Die Mischung wird in Gewebekulturflaschen abgefüllt und 4 - 5 Tage in feuchter Kammer ($CO_2$) inkubiert.

3) Herstellung der Gebrauchslösung zur Zellfusion.
2 μg Polymin D, 5 μg Na-Diamid oder 1 g Glutaraldehydlösung (25 %ig) werden in 30 ml RPMI-Medium, pH - 8,0 gelöst, autoklaviert und danach werden 3 ml/0,1 mM des Uridinisomers 5-β-D-Ribofuranosyluracil und 3 ml/0,1 mM Ribothymidin, kovalent an den Fluoreszenzfarbstoff A (1 μg/ml = nanogramm) gebunden, langsam zugegeben und auf pH - 7,5 eingestellt.

Ist eine in vivo Fluoreszenzmarkierung nicht vorgesehen, werden die löslichen Isomere der sRNA ohne Kopplung an den Fluoreszenzfarbstoff A oder B in der Fusion verarbeitet.

Fusionsvorgang

4) Alle Materialien aus der in vitro Immunisierung und der Zellfusion müssen steril und vor Gebrauch im Wasserbad auf 37°C erwärmt werden.
Die vorbereiteten Zell-Linien werden im Verhältnis 2:1 (Myelome, Zell-Linien : Blutzellenmaterial) in der Gebrauchslösung aus (3) vorsichtig vermischt und bei 500 g zentrifugiert. Der Überstand wird vorsichtig abgehoben und der Niederschlag vorsichtig von der Glasinnenwand abgeklopft. 2 ml Gebrauchslösung aus (3) werden innerhalb von 30 sec langsam zugegeben, der Röhrcheninhalt wird leicht gerührt, der Zellniederschlag mit sterilem Glasstab vorsichtig suspendiert, ca. 30 sec stehen gelassen und danach werden weitere 5 ml Gebrauchslösung aus (3) tropfenweise zugegeben. Maß läßt 30 sec stehen und gibt nochmal 5 ml Gebrauchslösung aus (3) zu und läßt 5 min stehen. Danach wird bei 500 g 5 min zentrifugiert und der Niederschlag vorsichtig im serumfreien Medium suspendiert (RPMI-Medium, serumfrei + 500 μl Polymin D oder 500 μl Na-Diamid oder 1 g Glutaraldehydlsg. 25%ig). Die Lösung wird in Zellkulturflaschen aufbewahrt. Bei Zugabe von Feederzellen (Makrophagen, etc.) vorzugsweise Lymphokine werden nur 2 ml Gebrauchslösung jeder Zentrifugation beigegeben.

Herstellung von Feeder-cells aus Blutzellen

5) Reagenzien: Ficoll 400 (Pharmacia)
Na-Benzylpenicillin
Streptomycinsulfat

11

Herstellung der Ficoll 400-Gebrauchslösung.

Zu 15 ml Ficoll 400 werden Na-Benzylpenicillin (20 bis 200 E/ml) und Streptomycinsulfat (10 bis 100 μg/ml) zugesetzt und eine halbe Stunde leicht gemischt. Diese Ficoll-Gebrauchslösung wird in sterilen Glasflaschen (spinner bottle) aufbewahrt.

Blutzellen aus Frischblut werden nach bekannten Verfahren (BONN, HARELL, BOND, J.Cell.Biol.36,369,1968) auszentrifugiert, gewaschen, zentrifugiert und Überstand abgesaugt. 4 ml einer Zellsuspension mit 4 ml NKM, pH-8,0 (0,153 mol/l NaCl, 5 mmol/l KCl, 5 mmol/l MgCl$_2$) verdünnen und mischen. Unter leichtem Rühren werden 80 μl Fluoreszenzfarbstoff A (1 μg/ml) zugegeben und 1 Std. bei 37°C inkubiert. Danach zentrifugieren (5 Min./500 Upm), Überstand verwenfen. 4 ml Zellsuspension mit 4 ml Gebrauchslösung (3) verdünnen, gut mischen und 1 Std. bei 37°C inkubieren, danach zentrifugieren, Überstand verwerfen. 4 ml Zellsuspension mit 4 ml NKM verdünnen und auf die Oberfläche des Gradienten (Ficoll 400, 10 ml) in der Rotorzelle (spinco 251-Rotor) aufbringen und bei 19.000 xg/90 Min. bei 5°C zentrifugieren. Alle Fraktionen aus den Gradienten mit einer Technicon-Dosierpumpe mit Glaskapillaren absaugen. Alle Fraktionen 4x mit NKM verdünnen, schütteln, absaugen. Letzte Verdünnung 5 Min./500 Upm zentrifugieren.

Fraktionsvolumen mit derselben Menge Ficoll 400 -Gebrauchslösung resuspendieren und in silikonisierten, magnetbetriebenen Rührflaschen (spinner bottle) aufbewahren.

Alle aus diesen erfindungsgemäßen Verfahren gewonnenen Antikörper werden bis auf eine 0,02 - 0,2 mg/ml Proteinkonzentration mittels Affinitätschromatographie mit oder ohne Protein A gereinigt und eingestellt.

Die klonale Selektierung und Reinigung der humanen monoklonalen Antikörper, Charakterisierung und Epitopenanalyse werden nach bereits bekannten Verfahren durchgeführt.

## Kopplungstechnik

Humane monoklonale Antikörper können wie folgt an Proteine, Hormone, Viren, Bakterien, Gene, Enzyme, DNA, RNA und deren Polymorphe, synthetische Schadstoffe, Allergene und andere biologischen Substanzen kovalent gebunden werden.

## Lösung A

10 μm Na-Diamid oder 5 μg Polymin D oer 1 g Glutaraldehydlösung 25%ig werden in 10 ml PBS-Puffer, pH -8,5 gelöst. Nach Zugabe von 5 mg Fluoreszenzfarbstoff A oder B (je nach Proteingröße) wird der pH-Wert auf 8,5 korrigiert und man rührt 30 min leicht.

Proteinverdünnung 1 und 2:

Proteinverdünnung 1 = 1 : 100/0,2 mg/ml Protein 10 μl Protein auf 1000 μl PBS-Puffer, pH - 8,5 (für Farbstoff B geeignet). Proteinverdünnung 2 = 1 : 1000/0,2 mg/ml Protein 1 : 1000 Verdünnung --- 100 μl auf 1000 μl PBS-Puffer,pH - 8,5 (für Farbstoff A geeignet)

Die Wahl des Farbstoffes A oder B hängt von der Endkonzentration der zu markierenden Substanz ab.

900 μl aus Proteinverdünnung 1 mit 900 μl Lösung A und 1 ml aus Proteinverdünnung 2 mit 1 ml Lösung A 1 min leicht rühren und 60 min bei Raumtemperatur stehen lassen. Danach pH-Wert auf 7,5 einstellen, über Nacht dialysieren (Puffer pH - 7,5) und über Säule Sephadex G -100 reinigen.

## Beispiel 7

Lymphozytenpräparation zum Einbau von humanen monoklonalen Antikörpern für die Transferfaktortherapie.

1) Blutzellmaterial, vorzugsweise Lymphozyten in Reagenzröhrchen 1x mit PBS-Puffer, pH - 7,2, 0,2 % BSA, 0,2 % NaN$_3$ waschen (0,4 x 10$^5$ Zellen in 2 ml Puffer) und 5 min bei 1000 Upm zentrifugieren.

2) humane monoklonale Antikörper (DNA/RNA-Kombinationen) oder andere Kombinationen je nach Spezifität 1:1000 bis 1: 1500 in 25 µl Portionen, vermischt mit 25 µl RPMI-Medium + 1 µg/ml Fluoreszenzfarbstoff A aliquotieren und bei Raumtemperatur 30 min inkubieren.

3) 2 x mit 2 ml PBS/BSA/NAN₃ waschen und bei 1000 Upm 5 min in der Eppendorfzentrifuge zentrifugieren.

4) 50 µl Fluoreszenzfarbstoff A (1 µg/ml) zum Niederschlag pipettieren und gut mit Vortex mischen, 30 min bei Raumtemperatur inkubieren.

5) 2x mit je 2 ml PBS/BSA/NAN₃ waschen, bei 1000 UpM 5 min zentrifugieren.

6) Zellen in 1 ml sterilem RPMI-Medium aufnehmen und kühl aufbewahren.

Alle für die Transferfaktortherapie oder zur Fusion benötigten Reagenzien sterilfiltern, autoklavieren und unter sterilen Bedingungen verarbeiten.

PBS/BSA/NaN₃

500 ml Endvolumen
500 ml PBS-Puffer (Bio-Merieux)
1 g BSA, reinst (Sigma)
1 g NaN₃ Natriumazid (Fluka)

Beispiel 8

Fluoreszenzmarkierung von opportunistischen Erregern mit weitgehendsten Organspezifitäten.

Nicht photochrome, menschenpathogene, schnell wachsende Mykobakterien und Mykoplasmen, z.B. Mykobakterium avium intracellulare oder Protozoen wie Pneumocystis carinii, die sehr häufig bei AIDS isoliert werden, vermehren sich als opportunistische Erreger in Makrophagen, dadurch kann virulentes Erregermaterial von avirulentem unterschieden werden.

Der fluorometrische Nachweis kann über die Anfärbung (in vivo oder chemisch) des Erregermaterials, oder über fluoreszenzmarkierte humane monoklonale Antikörper (hMAK) mit hoher Globulinkonzentration (humanes Antilymphozytenglobulin, Anti-D-Globulin) im direkten oder indirekten Immunfluoreszenztest mit dem Primärantikörper immunologisch, mikrobiologisch, histologisch im Serum, Bronchialsekret, lungenbioptischem Material und anderen biologischen Flüssigkeiten oder Gewebematerial durchgeführt werden.

Der bisher bekannte serologische Antikörpernachweis mit hyperimmunisiertem Kaninchenserum zeigt hohe Unsicherheiten. Diese führten zu Fehleinschätzungen und öfter wiederholenden Untersuchungen.

Es wird das Beispiel der Erreger-Färbung beschrieben.

Herstellung einer Nährboullion:

1000 ml Fleischwasser, 10 g Pepton, 5 g NaCl. Fleisch ohne Sehnen und Fett und Leber zerkleinern, mit doppelter Menge Wasser versetzen, ca. 100 ml irgend eines Serums (human) zugeben, verrühren und über Nacht bei 4° C stehen lassen, danach 30 min leicht sieden, abkühlen und filtrieren.

Im Pepton liegen die Proteine infolge tryptischer oder peptischer Verdauung als lösliche Peptide und Aminosäuren vor. NaCl dient als Osmostabilisator.

Gewinnung von Reinkulturen:

Manuelle Vereinzelung der Bakterienzellen, Züchtung eines Bakterien-Klons durch Überimpfung in flüssigem Medium.

Stoffaufnahme in das Bakterium:

100 ml Nährmedium wird mit 10 ml Fluoreszenzfarbstoff A oder B (1 µg/ml) (A bei z.B. Bakterien und B bei kleineren Bruchstücken) vermischt und der einzelne Bakterienklon wird dem Medium zugefügt. Vorteilhaft ist, nicht mehr als 50 Einzelzellen im Nährmedium zu suspendieren. Es werden verschließbare Reagenzröhrchen (max. 5 - 10 ml) verwendet.

Konservierung:

Die Kulturen werden in Röhrchen mit sterilem Paraffinöl überschichtet und kühl gelagert. Die Kulturen sind 6 Monate haltbar.

Nachweis von Mycobakterium in Biomassen:

Eine Menge von max. 5 ml wird der Ansatzlösung (Verfahrensbeginn) und dem gereinigtem Produkt (Verfahrensende) entnommen.

Ein biologisch eingefärbtes Mycobacterium (Einzelbacterium) wird in die Lösungen überimpft und bei 37° C 1 h inkubiert. Der aufgenommene Farbstoff wird freigesetzt und von evtl. vorhandenen Mycobakterien sofort aufgenommen.

Die schnelle Vermehrung von Mycobakterien, sichtbar im Mikroskop, Filter 360/blau, 420/grün-gelb, 520/rot, zeigt die Kontamination, bzw. das Vorhandensein von Mycobakterium an.

In entsprechender Weise können Protozoen, virulente, avirulente Mykobakterien und andere Parasiten zum Nachweis von Kontaminationen in komplizierten, schwer zu gewinnenden Natur- und anderen Produkten, oder mit fluoreszenzmarkierten humanen Antikörpern in biologischem Material fluoreszenzmarkiert werden.

Beispiel 9

Im folgenden ist ein BIAS (Biologisch-Immunologischer Analysen-Set)Kit für Bestimmungen im Blut, Plasma, Serum, Urin, Liquor oder anderen biologischen Flüssigkeiten beschrieben. Der BIAS-Kit wird zweckmäßig beispielsweise für jeweils 50 oder 100 Bestimmungen ausgelegt.

Grundsätzlich enthält ein solcher Kit/Liquid-Phase: fluoreszenzmarkierte humane monoklonale Antikörper, Peptide, DNA-RNA-Sonden oder deren Komponenten, Viren, Hormone und andere bekannte Bestandteile, wie die üblichen Lösungen Fluoreszenzstandard I und II

Pufferlösung pH - 7,5

Blockierungssubstanz (speziell aufbereitete sterile Molke)

Stoplösung pH - 9,0 (0,1 N NaOH)

Der BIAS Kit/Solid-Phase wird lediglich durch 1 Mikrotiterplatte (95 Vertiefungen) und Waschlösung (PBS-Puffer + Tween 20/ 2%ig) ergänzt.

Bei BIAS in vivo Kit besteht der Inhalt des Testbestecks aus fluoreszenzmarkierten humanen monoklonalen Antikörpern, antigenfixierten Tüpfelplatten oder Objektträgern, Papier-oder Kunststoffstreifen mit Rührstäbchen, Waschlösung, der BIAS in vivo screening Kit beinhaltet lediglich antigenfixierte Objektträger und Waschlösung (PBS-Puffer + Tween 20/2%ig).

Der BIAS-Kit/Liquid-Phase kann fluorometrisch oder photometrisch,

der BIAS-Kit/Solid-Phase kann fluorometrisch, photometrisch, mikroskopisch oder mit Fluoreszenzlampe,

der BIAS in vivo - und der BIAS in vivo screening Kit können mikroskopisch oder mit UV-Lampe bestimmt werden.

**Ansprüche**

1. Verfahren zur Diagnose von erblichen und immunologischen Erkrankungen durch Fluoreszenzmessung biologischer Substanzen, insbesondere von Viren, Antikörpern, Antigenen, Proteinen, Peptiden, Polynukleotiden, Enzymen, Haptenen, DNAs und RNAs, dadurch gekennzeichnet, daß man die zu diagnostizierende Substanz, wenn ihr Molekulargewicht über ca. 5000 liegt, mit dem Kondensationsprodukt von Flavoprotein, insbesondere Riboflavin-5′-monophosphatnatriumsalz mit Alpha-Liponsäure, das nach Zugabe von Jodacetamid und Dialysieren nach Destillation zurückbleibt, markiert bzw. die zu diagonstizierende Substanz, wenn ihr Molekulargewicht unter ca. 5000 liegt, mit dem weiteren Umsetzungsprodukt des Destillationsrückstandes mit einer Reaktionslösung aus 1,4-Bis(4-methyl-5-phenyl-2-oxazolyl)-benzol und p-

14

Terphenyl in einem organischen Lösungsmittel, dem Maleinsäureanhydrid zugesetzt ist, und das nach mehrstündigem Stehen auf ein etwa neutrales pH eingestellt und in üblicher Weise gereinigt ist, markiert und dann in üblicher Weise, ggfs. nach Abtrennung, die Fluoreszenzmessung vornimmt oder die zu untersuchende biologische Substanz mit der korrespondierenden markierten Substanz, ggfs. unter Freisetzung des Farbstoffes und ggfs. Übertragung des Farbstoffes auf die korrespondierende Substanz versetzt und dann, ggfs. nach biochemisch üblichem Inkubieren, die Messung durchführt.

2. Verfahren nach Anspruch 1, dadurch gekennzeichnet, daß man als zu markierende Proteine humane monoklonale Antikörper oder Anti-Antikörper oder deren DNA und/oder RNA oder Fragmente davon einsetzt.

3. Verfahren nach Anspruch 2, dadurch gekennzeichnet, daß man die zu markierenden Substanzen in geklonter Form, ggfs. als geklonte Fragmente, einsetzt.

4. Mittel zur Durchführung des Verfahrens nach Anspruch 1 bis 3 sowie zur Therapie, dadurch gekennzeichnet, daß sie gemäß Anspruch 1 markiert und ggfs. in an sich üblicher Weise isoliert und ggfs. in Zellen eingebaut sind.

5. Mittel nach Anspruch 4, in Form von in lebendem Material marktierten Antikörpern.

6. Mittel nach Anspruch 4, in Form von in vitro markierten Antikörpern für weniger sensitive Routinebestimmungen oder in Form von in lebendem Material, jedoch im Reagenzglas durch Fusionierung erhaltenen markierten Antikörpern und Zellen für die Ermittlung von Bindungsfähigkeit in vivo und insbesondere zur Tumorfrüherkennung.

7. Verwendung der Mittel nach Anspruch 4 oder 5 zur Therapie von immunologischen Erkrankungen durch Bestrahlung der in Lymphozyten eingebauten, fluoreszenzmarkierten biologischen Substanz mit Licht entsprechender Wellenlänge zur Auslösung der Fluoreszenz zur Inaktivierung des Antigens und Transferfaktor Therapie.

8. Kit zur Durchführung des Verfahrens nach Anspruch 1 bis 3, enthaltend

a) für in vitro Liquid-Phase:

Fluoreszenzmarkierte humane monoklonale Antikörper, die der zu bestimmenden Substanz korrelierende Substanz, wie Peptide, DNA-, RNA-Sonden oder deren Komponenten, wie Viren, Hormone Fluoreszenzstandard I und II

Pufferlösung pH 7,5

Blockierungssubstanz

Stoplösung pH 9

b) für in vitro Solid-Phase zusätzlich eine Miktrotiterplatte und Waschlösung

c) für BIAS in vivo Kit:

Fluoreszenzmarkierte humane monoklonale Antikörper,

antigenfixierte Tüpfelplatten oder Objektträger,

Papier- oder Kunststoffstreifen

Rührstäbchen,

Waschpuffer

d) für BIAS in vivo screening Kit:

antigenfixierte Objektträger

Waschpuffer